# EUROPEAN PATENT SPECIFICATION

(11) **EP 0 601 130 B1**
(45) Date of publication and mention of the grant of the patent: **26.08.1998**
(21) Application number: 92923298.1
(22) Date of filing: 28.10.1992
(51) Int. Cl.: A45D 26/00

(54) **HAIR REMOVAL METHOD**
VERFAHREN ZUM ENTHAAREN
PROCEDE D'EPILATION

(30) Priority: 29.10.1991 US 783789
(43) Date of publication of application: 15.06.1994
(60) Divisional application: 98201450.8
(73) Proprietor: THERMOLASE CORPORATION, San Diego CA 92121-4339 (US)
(72) Inventor: TANKOVICH, Nikolai, I., San Diego, CA 92121-2305 (US)
(74) Representative: Hirsch, Marc-Roger
(86) International application number: PCT/US92/09189
(87) International publication number: WO 93/08715

(56) References cited:
- WO-A-86/02783
- WO-A-91/04073
- DE-A- 3 220 962
- US-A- 3 693 623
- US-A- 3 769 963
- US-A- 3 794 028
- US-A- 3 834 391
- US-A- 3 900 034
- US-A- 4 388 924
- US-A- 4 461 294
- US-A- 4 608 978
- US-A- 5 059 192
- LASERS IN SURGERY AND MEDICINE, vol.10, no.2, 1990, NEW YORK US pages 189 - 193, XP385876 FINKELSTEIN AND BLATSTEIN 'EPILATION OF HAIR-BEARING URETHRAL GRAFTS'
- 1984- ANDREONI et al., "Porphyrinsins in Tumor Phototherapy", pages 143-155.
- 20 June 1977 ANDERS et al., "Investigation and Therapy In Dermatology With Dye Lasers", pp. 520-526.

## Description

This invention relates to apparatus and methods for hair removal.

The principal methods presently used for hair removal involve the use of electrolysis techniques or chemical depilatories. These techniques involve some pain, are time consuming and demand a fair degree of expertise in their application and normally do not guarantee a permanent effect.

Laser use in medicine is well known. For example, lasers are used in surgery for both cutting and cauterization. Lasers have been used for many years for removing tattoos under the surface of the skin. In this case a laser beam penetrates the skin and is absorbed by and destroys the ink particle. A similar procedure has been used for years to remove birth marks where the laser is matched to an absorption peak of the erythrocyte's hemoglobin in the tiny capillaries under the skin to destroy the capillaries.

Medical researchers have experimented with using photoactive dyes to enhance light treatment of tissue. Anders *et al* describes *in vivo* experiments for determining benefits of applying a photosensitizing dye to a section of skin prior to photodynamic therapy of special dermatosis and for tumour destruction (Investigation and Therapy in Dermatology with Dye Lasers, Laser 77 Opto-Electronics: Conference Proceedings, ed. W. Waidelich, Munich, 20/24 June, 1977, pg. 520-26). According to this reference, the dye thioropyronine was first applied to epidermal cells and the dyed epidermal cells were then irradiated with laser light at wavelengths of 574.8 nm and 514.5 nm. The light absorption properties of the irradiated cells were measured with a second, test laser, and the results compared with similar measurements on untreated cells.

Ambesi-Impiombato *et al* describe *in vitro* cell culture experiments in which application of a photoactive dye, Hematoporphyrin-Derivative (HpD), to a cell culture and then irradiation of the culture with laser light was found preferentially effective for treatment of tumour-bearing cells (Effects of HpD and Laser on Transformed and Corresponding Normal Cultured Cells: Differential Cytotoxicity as an *In Vitro* Model for Tumour Photochemotherapy, Porphyrins in Tumour Phototherapy, ed. A. Andreoni and R. Cubeddu, 1984, pg. 143-55).

It has been known for at least 20 years in the medical profession that selective absorption of laser radiation can sometimes be enhanced by the technique of staining pathological tissues with various vital dyes. (See Goldman US Patent No: 3 769 963).

The present invention is not concerned with any medical or surgical procedure, but with hair removal for essentially cosmetic and like purposes.

The prior art of hair removal also includes attempts at removing hair with laser beams. Three such techniques are described in the following United States patents: Weissman et al., Method for Laser Depilation Device and Method, Patent No. 4,388,924; Sutton, Depilation Device and Method, Patent No. 4,617,926; and Mayer, Depilation by Means of Laser Energy, Patent No. 3,538,919. All of these devices and methods teach the removal of hairs one hair at a time with a narrowly focused laser beam. Therefore, they are relatively inefficient and time consuming. A recent patent by Zaias, Patent No. 5,059,192 issued 10/22/91 discloses a process for using a laser beam matched to the melanin found at the base of the hair follicle and papilla.

What is needed is a simple, harmless device and method for removal of hair over a relatively broad area of skin, for cosmetic and like purposes.

According to the invention, there is provided a process for the removal, from a section of human skin, other than in the course of a surgical procedure, of hairs growing in hair ducts from follicles at the bottom of said ducts and being nourished by skin tissue immediately surrounding said follicles, essentially without damage to skin tissue except to said skin tissue immediately surrounding said follicles comprising the steps of:
a) selecting a contaminant having an optical absorption of at least one frequency band of light which will penetrate said section of skin,
b) applying said contaminant to said section of skin in such a manner for some portion of said contaminant to infiltrate said hair ducts, and
c) illuminating a spot of said section of skin containing contaminated hair ducts with said at least one frequency band of light, a significant portion of which penetrates the skin and is absorbed in said contaminant in said hair ducts, said optical absorption of said contaminant at said at least one frequency band of light being high enough to cause a reaction destroying said follicles or damaging the skin tissue feeding said follicles.

Preferred embodiments may include one or more of the following additional features. The high absorption at the at least one frequency is a resonance peak for the contaminant. The contaminant is comprised of an oil and an absorber, preferably carbon powder, suspended in the oil. Tissue surrounding the plurality of hairs is damaged in order to cause death of the follicles over a period of a few days. The contaminant is a hair dye. Preferably, the frequency band of light is produced by a laser, e.g. the frequency band of light is a band centred about 10.6 microns (wavelength) and is produced by a CO₂ laser. The at least one frequency band of light comprises light at a wavelength of about 1.06 microns. Preferably, the frequency band is matched to an absorption peak in the hair dye. More preferably, the hair dye is standard commercial hair dye #124 and frequency band is produced by a 587 nm dye laser. The contaminant is a photosensitizer and the frequency band is matched to resonance peaks in the photosensitizer. The contaminant and at least one frequency band of light are chosen from the following group of contaminant-laser combinations:
Hematoporphyrin Derivation with Argon Dye Laser (632 nm)
Indocyanine Green with Diode Laser (785 nm)
Microcyanine with Copper Vapor Laser (540 nm)
Photophrin II with Argon Dye Laser (630 nm)
Chlorin-E6 with Dye Laser (660 nm)
Chlorophyll Derivatives with Argon Dye Laser (630 nm)
Black Ink with Ruby Laser (694 nm)
Carbon Powder in Oil with Co₂ Laser (10.6 microns)
Carbon powder in oil with Nd:YAG Laser (1.6nm).

Any of the above with Tunable titanium-sapphire Laser.
The at least one frequency band of light comprises light at a wavelength of about 1.06 microns. The contaminant comprises particles, preferably having a size of about 10-20 nm, suspended in a medium. The contaminant is a material which is not readily absorbed by the follicles or surrounding tissue, and, preferably, the reaction which destroys the hairs by causing death of the follicles is due to indirect conductive heating of the follicles or of the skin tissue feeding the follicles in the vicinity of the contaminant. The illuminating step is carried out while observing the section of skin undergoing illumination and continuing the illuminating at least until the hair on the section of skin begins to curl.

It will thus be seen that we provide a means for the removal of unwanted human hair. The hair or the skin tissue feeding or surrounding the hair on a section of skin is contaminated with a substance having a high absorption of a frequency band of light. The section of skin is illuminated with light at sufficient intensity and duration to kill the follicle or the skin tissue feeding the hair. Specific embodiments produce death of the follicle or the tissue by heating or by photochemical reaction.

In the drawings:-
FIG. 1 is a drawing of a section of human skin showing a growing hair.
FIGS. 2 A, B and C show a cross section of skin and 3 hairs during 3 stages of a process of one embodiment of the present invention.
FIG. 3 shows qualitatively the paths of the photons of a laser pulse showing absorption in a carbon-oil suspension.
FIG. 4 A and B show the temperature distribution near a typical hair during the process of a preferred embodiment of the present invention.
FIG. 5 shows qualitatively the paths of the photons of a laser pulse showing absorption in hair dye.

Preferred embodiments are described below by reference to the figures.

### COAT AND HEAT METHOD

### Skin Preparation

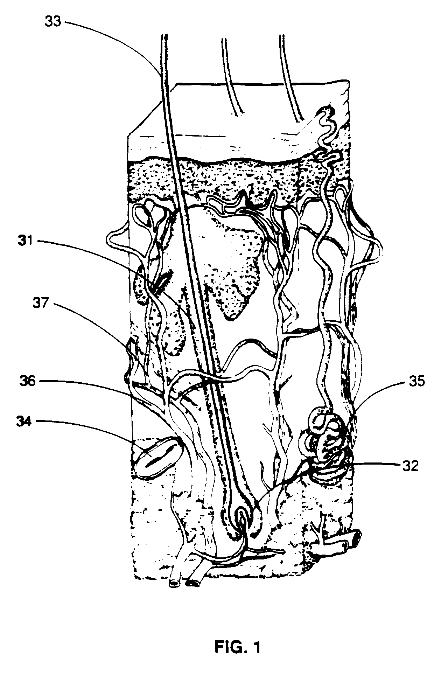

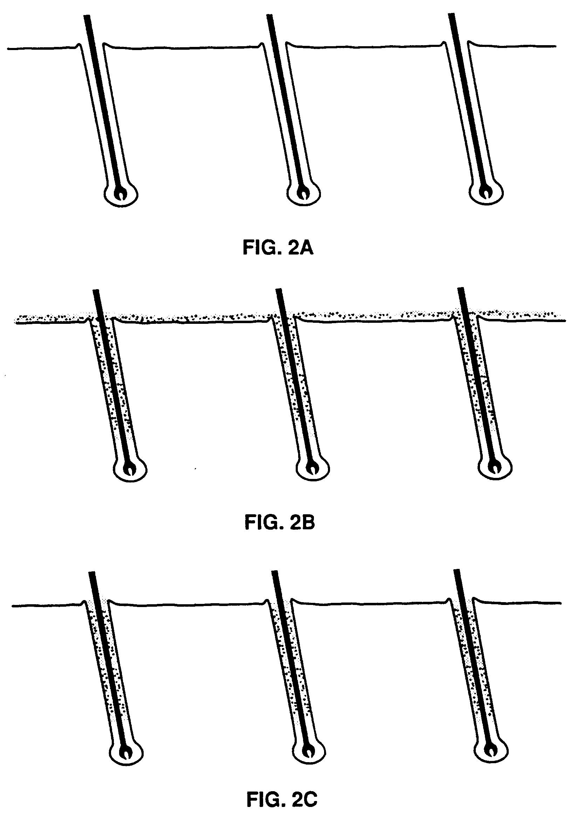

A section of human skin showing a cross-section of one hair is shown in Fig. 1. A first preferred embodiment is described by reference to Figs. 2 - 4. Fig. 1 shows a hair duct 31, a follicle 32 at the bottom of the duct, a hair shaft 33, a nerve ending 34, a sweat gland 35 and arteries 36 and veins 37. First, a laser absorbing carbon suspension is prepared of carbon powder in peach oil. The particle size of the powder preferably is about 10-20 nm and its concentration preferably is about 15% to 20% by volume.

A clean section of skin is depicted in FIG. 2A. This suspension is rubbed on the skin with a massaging action so that portions of the carbon suspension infiltrate the hair ducts of the hair that is to be removed as shown in FIG. 2B. Ultrasound with frequencies in the range of 3.5 to 10 MHz at a power level of about 0.1 to 0.2 with 4 to 5 minutes could be used to help force the suspension into the ducts. Next the surface of the skin is cleaned preferably with an alcohol pad to make the skin surface clean but to leave the hair pores contaminated with the carbon suspension as shown in FIG. 2C.

### Laser Application

The laser device used in this preferred embodiment is a C0₂ pulse laser which has the spikes in the range of 10.6 microns. Light in this range will pass through the outer layer of the surface of the skin and is readily absorbed in carbon. Laser parameters such as pulse width and repetition rate can be selected to best fit the skin and hair types of the human subjects. The parameter for two specific examples which we have utilized with good results for hair removal are shown in Table 1:

**Table 1**

| **Parameters Preferred.** | | |
|---|---|---|
| | **First Example** | **Second Example** |
| Pulse Width | 275 ns | 200 ns |
| Repetition Rate | 30 Hz | 8 Hz |
| Laser Spot Size | 1 cm² | 1 cm² |
| Energy per Pulse | 0.1 Joule | 0.2 Joule |
| Scanning Rate | 20 seconds per 10 cm² | 30 seconds per 10 cm² |

Each point on the skin receives illumination for about 2 seconds and each square centimeter receives about 6 Joules. Some of the light is reflected. Of the light which is not reflected, a significant portion of the energy of each pulse is absorbed in the carbon.

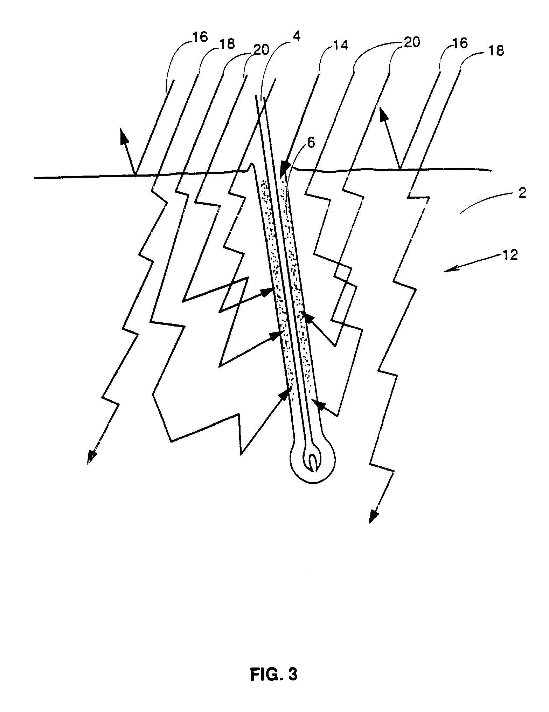

FIG. 3 shows a simplified view of a section of human skin and qualitatively the paths 12 of some of the photons of a laser pulse illuminating a section of skin 2 containing a hair duct with a hair 4 contaminated with carbon suspension 6. A few of the photons travel directly through the skin and are absorbed in the carbon (depicted by photon 14). Some are reflected from the skin surface (depicted by photons 16). Some are absorbed in the skin (depicted as photons 18) and a portion of the photons are absorbed in the carbon.

Operating within the parameters specified is important. They have been chosen to preferentially heat the carbon suspension which in turn heats the hair follicles and the blood vessels feeding the follicles to temperatures high enough to kill the hair follicles and/or the tissue feeding the follicles but to minimize the heat to the rest of the skin tissue. The energy application time is a most important parameter. It must be chosen so that a large amount of energy is deposited in the suspension quickly so that the temperature of the suspension rises rapidly to about above 70 - 80°C. This temperature applied for about 1 second is high enough to kill the follicles and/or the vessels feeding the follicles. During this short period heat transferred to the skin tissue is not enough to damage the skin tissue except that tissue immediately surrounding the follicle. A good practice is to start out with the power densities specified. It will be evident when enough energy is being supplied because the hair shaft will begin to curl. If curling is not achieved the power density could be increased up to about 2-3 Joules per square centimeter or until sufficient energy is delivered to deutilize the hair.

We have performed hair removal experiments using the parameters shown in Table 2 with excellent results. There is no significant pain. The hair is removed and there is no apparent detrimental effect.

We performed a qualitative mathematical analysis in order to estimate heart absorption and temperature distribution in the hair and skin tissue. This analysis is shown in Table 3.

Thus, under these assumptions each pulse would heat the carbon oil suspension roughly about 5°C. (The reader is cautioned that the above analysis is not to be relied on as a quantitative description of the process of heating the carbon oil suspension in the hair duct. For example, for many people the assumption that 1/4 of the energy of each pulse goes into the hair duct is probably too high.)

Each pulse will also heat the skin in general. We do not have a good estimate of the portions of the energy of the pulse reflected, absorbed in the hair ducts and absorbed in the skin in general. However, we have assumed for this qualitative analysis that about 1/2 of the energy the laser pulse reflects, 1/4 is absorbed in the hair ducts and 1/4 is absorbed in the skin in general. If we assume that the skin is heated fairly uniformly to a depth of 0.2 cm, a skin density of 1 gm/cm³ and a specific heat for the skin, of 4 J/gm°C the 0.025 J pulse will heat this typical skin section

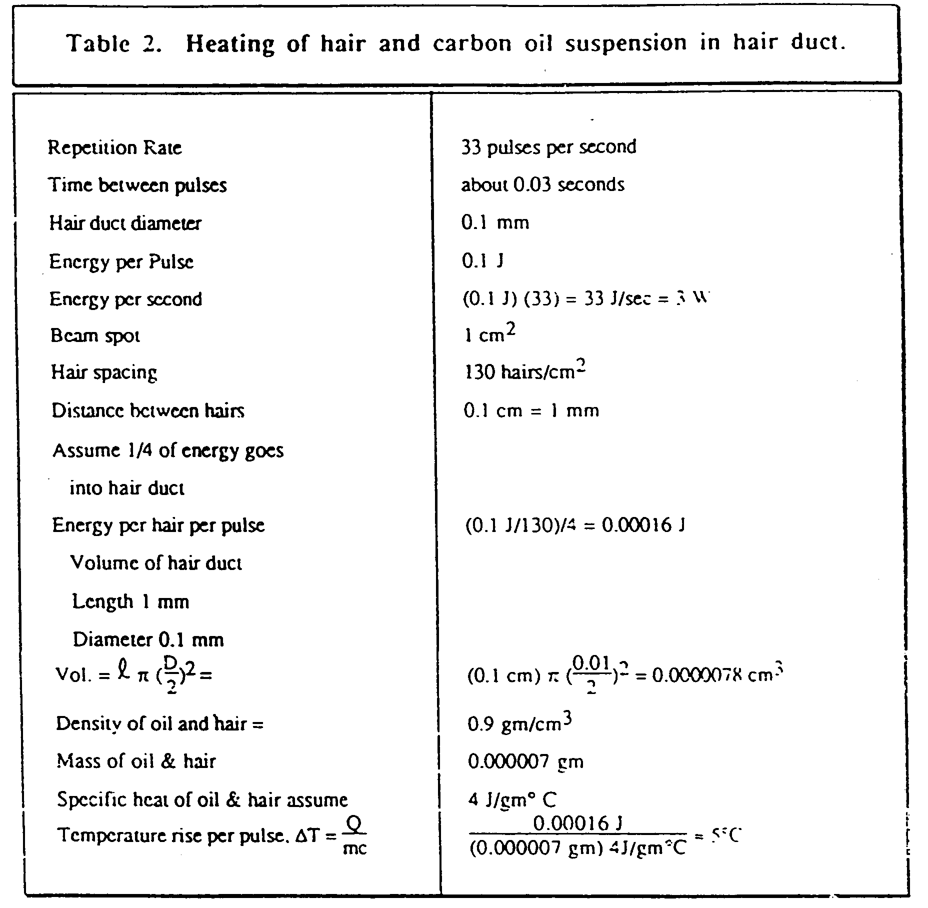

about 0.04 degrees C. Based on these assumptions, the 60 pulses over about 2 seconds will give a general heating of about 2°C. Therefore, heat deposited generally to the skin would be negligible. (Again, the reader is cautioned regarding the qualitative nature of this analysis.

In practice we believe much of the energy from the pulse CO₂ laser is absorbed in a very thin area of the surface possibly as thin as 0.1 mm depending on the dryness of the skin. In some cases a very thin layer of the skin is actually vaporized in the process, but this is usually the layer which consists of essentially dead cells which naturally flake off the skin surface. Also, since the epidermis is such a poor heat conductor the underlying layers of skin is typically protected from damaged except those portions very close to the carbon oil suspension.)

However, heat from the hot carbon oil suspension will be transferred by conduction to the tissue surrounding the hair duct. We used the following relationship (see note 10 of Zwig & Wibber, IEEE Journal of Quantum Electronics, Vol. QE-23, No. 10 Oct. (1987). Mechanical and Thermal Parameters In Pulsed Laser Cutting of Tissue) to estimate the heat spread from the hot carbon oil suspension in the duct:$\text{δ = /Kτ}$ where δ represents the thickness of a heated zone during a time τ, K being the heat of conduction. Assuming K = 1.44 x 10⁻³ cm²/S and using 0.03 sec as the time interval between pulses, we estimate that the heat spreads out by about 0.007 cm from the hair duct between each pulse. This is about equal to the radius of the hair duct so we assume that about one half of the temperature rise from each pulse is transferred to the surrounding tissue during the 0.03 second following each pulse. This means that the net increase in the temperature of the carbon-oil suspension from each pulse will be roughly 2.5°C.

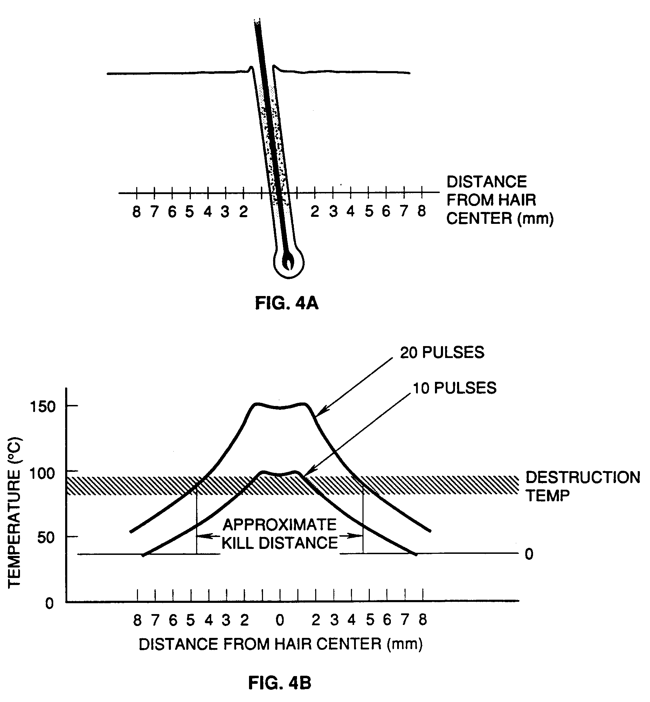

Thus, as depicted in FIG. 4 in about 2/3 second the temperature of the carbon-oil suspension in the hair duct has risen from a normal temperature of 37°C to about 90°C, a temperature high enough to kill the follicle and the tissue cells immediately surrounding the hair follicle (i.e., within about ± 5 hair diameter). In a little more than one second the temperature has risen to about 140°C which we currently propose as the upper range.

At this point the person concerned would begin to feel pain. Therefore, the illumination should be applied so that no spot is illuminated longer than about one or two seconds during one scan. FIGS. 4A and 4B shows a rough approximation of the temperature distribution between ± 8 millimeters of the center for a typical hair duct after 20 and 40 pulses.

For this process I illuminate a 10 cm² area by making 2 or 3 passes over each spot during a 20 second scanning period. For each spot the temperature will have dropped from the range of about 100°C - 140°C to below about 50°C during the approximately 7 seconds between scans.

As a result of the illumination, we estimate that for many people essentially all follicles will be killed or will die within 2 weeks because of reduced nourishment due to the destruction of the tissue surrounding the hair duct which feed the follicle. We also estimate that the destroyed tissue is confined to within about 3-6 millimetres (about 6-12 hair diameters) of the centre of the hair. Although we list this as a preferred embodiment, it does not work well on all persons. In some cases pain and some surface burning is experienced before the hair tissue is destroyed. For these persons, one of our alternative embodiments is recommended.

### NEAR INFRARED LASER METHOD

This process is the same as the first embodiment described above except the laser wavelength is 1.06 microns, the pulse duration is about 1000 times less (i.e, in the range of 25-30 pico seconds), the energy per pulse is about 100 times less or about 3-6 mJ and the spot size is about 0.1 to 0.3 cm². At this wavelength the skin penetration is maximum. In this case much less energy is required because a much larger percentage of the energy is absorbed in the contaminant.

### STAIN METHOD

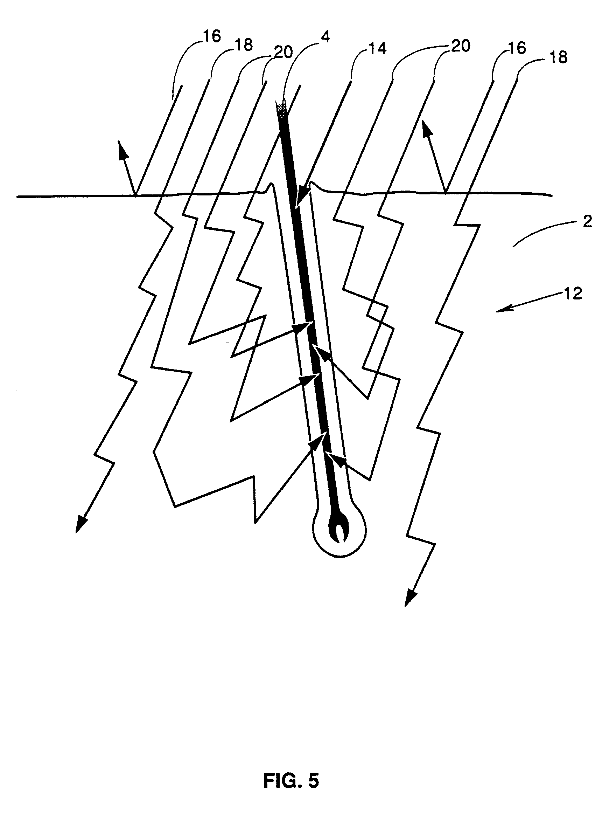

A second embodiment involves the use of dyes to stain the hair follicles. A pulse laser beam of light having a wavelength corresponding precisely to a resonance frequency of the dye illuminates the hair and skin area where the hair is to be removed. The dye and laser beam are chosen so that there is very little absorption by the skin tissue but great absorption by the dye. As indicated in FIG. 5 the photons will undergo diffuse reflection in the photon intersects the hair it is absorbed.

To stain the follicles, dye is mixed to form a solution which will penetrate into the follicles. A good substance used to form this solution is hydropertis. In one embodiment commercial hair dye #124 (deep black with blue) and India ink which already contains such a solution. It is rubbed on the skin and hair and let stand for 30 minutes The dye will migrate through the hair all the way to the root. India ink could also be used.

The skin is cleaned using standard dye removal solution. This India ink and dye #124 have an absorption peaks at ∼ 694 nm and ∼ 587 nm which matches perfectly with the wavelength of 587 nm dye laser. Dye #124 also has a resonance of 531 and 584 nm corresponding to the output of a copper vapour laser supplied by Spectra Physics.

For this embodiment we use a pulse width of 150 ns ruby laser and 200 µs dye laser. With a beam cross section diameter of 0.4 cm, the energy density is 2.5 - 8.5 J/cm². There are many other dye-laser combinations available which will be obvious to persons skilled in the laser art.

The secret is to match the laser wavelength with a resonance peak in a dye which can be applied to and absorbed in the follicles. India ink (essentially the same as tattoo ink) has a high absorption from UV up to IR.

We describe below a good general procedure for hair removal practising the stain method.
1. Discolour hairs with hydroperoxide 1 hour prior to staining hairs.
2. Cut or shave hairs leaving about 1 mm of hair above the skin.
3. Stain hairs with the ink or dye (red or orange, preferably). More ink or dye would be located around the hair and its pores because of the liquid surface tension near the hair.
4. Leave substance covered for 40-50 minutes.
5. Wash skin surface several times with alcohol, until the skin surface returns to its normal colour, except hair pores.
6. Make 3-4 spots for the test with different power densities to choose individual optimal dose for the human subject.
7. Start lasering in 3-6 hours after the staining procedure, one laser shot per spot.
8. Cover the area irradiated with Aloe Vera Gel or Laser Cream after the procedure.
9. Give these instructions to the human subject:
   - use Bicicytrine ointment topically first three days;
   - spare the area irradiated when taking shower, don't use hard sponges;
   - protect the area from direct sunlight by sunscreen or dress;
   - take Tylenol if there is any discomfort;
   - call if necessary.
10. Examine the skin in 1, 2 and 3 weeks.
11. Repeat the procedure if necesary for the hairs which were in Anagen or Caragen phases during the laser HR.

Titanium-Sapphire laser could be used. This laser covers the parameters of Ruby laser, penetrates human skin about as well as the Ruby laser and has a wider band of radiation within the absorption spectrum of these dyes.

### PHOTO CHEMICAL DESTRUCTION

A third embodiment for practising this invention is to apply a photosensitizer to the hair so that it is absorbed along the full length of the hair to the root. The skin area is then illuminated with laser light which readily penetrates the skin but is absorbed resonantly by the photosensitizer. The photosensitizer undergoes a chemical reaction which is deadly to the hair follicles.

A good specific example of this embodiment of the present invention is to apply a 20% solution of hematoporphyrin derivatives topically to the skin over where the hair to be removed has been recently shaved. The solution is absorbed into the portion of the hair remaining under the skin by capillary action. The skin is then cleaned thoroughly with an alcohol pad. Next the skin area is illuminated with an argon dye laser at 632 nm. The energy required is about 5-10 Joules per square centimeter. In this case, the time period is not very important. It could be several minutes per square when the laser energy is absorbed in the hematoporphyrin derivatives, singlet oxygen is produced as a result of photochemical reaction. The singlet oxygen is toxic for protein and phosphorlipids in the hair follicles and the follicles are thus killed.

### SKIN COVER METHOD

This method is essentially the same as the Coat and Heat Method described above except that the surface of the skin is not cleaned after the application of and massaging of the carbon-oil suspension. The skin surface appears like that shown in cross-section in FiG. 2B instead of 2C for the irradiation step. In this case the carbon-oil suspension serves as a shield for the skin surface preventing the CO₂ laser light from penetrating the skin to any significant extent. The carbon oil is heated in the process to very high temperatures for very short periods of time. Heat is transformed by conduction down the hair ducts through the carbon-oil suspension to kill the tissue immediately surrounding the hair. The outermost surface of the skin being a very good insulator prevents any substantial heat transfers to the lower layers of the skin and prevents any significant damage to the skin. The outermost cells of the skin usually flakes off after the procedure but his is a normal process and is completely harmless.

### ORAL AND INTRAVENOUS CONTAMINATION OF HAIR OR TISSUE FEEDING THE HAIR

It is also possible to contaminate orally or intravenously the hair or tissue feeding the hair. A preferred method for oral contamination is as follows:

A solution of disodium fluoresein 2-5% concentration given orally. Within 3 to 72 hours a significant portion of the disodium fluoresein will be concentrated in the body hair of the human subject. Sections of the skin containing the hair to be removed is irradiated with a laser pulsed at a wavelength matched to NaFl. Preferred laser sources are HeCd (441 nm), Nd:YAG (1.064 nm) frequency shifted to about 500 nm and Er:Glass (1.54 µs) tripled to 513 nm. Other sources with wavelengths from 370 nm to 520 nm would be satisfactory. Preferred power levels are between 5 to 15 J/cm² depending on hair depth, type of skin, Disodium Fluoresein metabolism, etc. Preferred pulse duration is 1µs or less.

### OTHER CONTAMINANT - LASER COMBINATIONS

There are many other chemicals which can be used in the stain method and the photochemical method. We have listed in Table 3 some of these along with a corresponding recommended laser for the illumination.

### OTHER EMBODIMENTS

It is very important for all of these embodiments and in other embodiments which will be apparent to persons skilled in the art that the light absorbing substances have a very high absorption coefficient at frequencies which pass readily through the surface of the human skin. An illumination source is matched to this frequency. The substance used can be one with a high resonance peak at the frequency or it can be one with a high broad absorption coefficient over a wide band continuing the illumination frequency. The important thing is to use a light of a frequency which diffuses through the skin and has a relatively low absorption in the skin and to use an absorber for contaminating the hair which will provide very high absorption of the light. Persons skilled in the art will recognize that certain frequencies will be preferred for light skinned persons and other frequencies may be preferred for dark skinned persons. The preferred beam size is about 1 square centimeter but could be as large as about 5 square centimeters.

**TABLE 3**

| **Dyes and matching laser.** | |
|---|---|
| **DYE** | **LASER** |
| Hematoporphyrin derivatives | Argon Dye (630 nm) |
| Indocyanine Green | Diode Laser (785 nm) |
| Microcyanine | Copper Vapour (540nm) |
| Photophrin II | Argon Dye (630 nm) |
| Chlorin -E6 | Dye (660nm) |
| Chlorophyll derivatives | Argon Dye (630 nm) |
| Black Ink | Ruby Laser (694 nm) |
| Any of the above | Tunable titanium-sapphire |

## Claims

1. A process for the removal, from a section of human skin, other than in the course of a surgical procedure, of hairs growing in hair ducts from follicles at the bottom of said ducts and being nourished by skin tissue immediately surrounding said follicles, essentially without damage to skin tissue except to said skin tissue immediately surrounding said follicles comprising the steps of:
a) selecting a contaminant having an optical absorption of at least one frequency band of light which will penetrate said section of skin,
b) applying said contaminant to said section of skin in such a manner for some portion of said contaminant to infiltrate said hair ducts, and
c) illuminating a spot of said section of skin containing contaminated hair ducts with said at least one frequency band of light, a significant portion of which penetrates the skin and is absorbed in said contaminant in said hair ducts, said optical absorption of said contaminant at said at least one frequency band of light being high enough to cause a reaction destroying said follicles or damaging the skin tissue feeding said follicles.

2. A process according to claim 1, wherein said optical absorption at said at least one frequency is a resonance peak for said contaminant.

3. A process according to claim 1, wherein said contaminant is a material which is not readily absorbed by the follicles or surrounding tissue.

4. A process according to claim 1, wherein said contaminant comprises particles suspended in a medium.

5. A process according to claim 4, wherein said particles have a size of about 10-20 nm.

6. A process according to claim 4, wherein said contaminant is comprised of an oil and an absorber suspended in said oil.

7. A process according to claim 6, wherein said absorber is carbon powder.

8. A process according to claim 6, wherein said oil is peach oil.

9. A process according to any of claims 1 to 4; wherein tissue surrounding said hairs is damaged in order to cause destruction of the follicles over a period of a few days.

10. A process according to any of claims 1 to 4, wherein said at least one frequency band of light is produced by a laser.

11. A process according to claim 10, wherein said at least one frequency band of light comprises light at a wavelength of about 1.06 microns.

12. A process according to claim 10, wherein said light is produced by a near infrared laser.

13. A process according to claim 10, wherein said at least one frequency band of light is a band centred about 10.6 microns (wavelength) and is produced by a CO₂ laser.

14. A process according to any of claims 1 to 4, wherein said illuminating step is carried out while observing the spot of skin undergoing illumination and continuing said illuminating at least until hairs on the spot of skin begin to curl.

15. A process according to any of claims 1 to 4, wherein said reaction heats the follicles or heats the skin tissue feeding said follicles in the vicinity of the contaminant by indirect conductive heating.

16. A process according to any of claims 1 to 4 or 10, wherein said spot has an area of about 1-5 square centimetres.

17. A process according to any of claims 1 to 4, wherein applying said contaminant includes rubbing said contaminant on said section of skin.

18. A process according to any of claims 1 to 4, wherein applying said contaminant includes applying ultrasound to the contaminant on the skin.

19. A process according to any of claims 1 to 4, wherein applying said contaminant including forcing the contaminant into the ducts.

20. A process according to any of claims 1, 2, 10 or 16, wherein said contaminant comprises a dye.

21. A process according to claim 20, wherein said dye is capable of staining the follicles.

22. A process according to any of claims 20 or 21, wherein said at least one frequency band of light is matched to an absorption peak in said dye.

23. A process according to any of claims 20, 21 or 22, wherein said dye comprises a commercial hair dye.

24. A process according to any of claims 20 to 23, wherein said dye comprises india ink.

25. A process according to any of claims 20 to 23, wherein said at least one frequency band comprises an intensity peak at a wavelength of at least one of about 694 nm, about 587 nm, 584 nm and 531 nm.

26. A process according to any of claims 1 to 4 or 20 to 25, further comprising cleaning said section of skin after applying said contaminant and prior to illuminating.

27. A process according to any of claims 1 to 4, or 20 to 25, wherein said illuminating includes illuminating with pulses of laser light.

## Patentansprüche

1. Ein Verfahren zur Haarentfernung von einem Bereich der menschlichen Haut, außerhalb der Durchführung einer chirurgischen Maßnahme, wobei die Haare in Haarkanälen aus Follikel wachsen, welche sich im Boden der Kanäle befinden und von dem Hautgewebe genährt werden, das die Follikel unmittelbar umgibt, im wesentlichen ohne Schaden des Hautgewebes, außer dem Hautgewebe, das die Follikel unmittelbar umgibt, umfassend die Schritte :
(a) Auswahl einer Fremdsubstanz, die eine optische Absorption von Licht mindestens eines Frequenzbereichs zeigt, die in der Lage ist, in den Hautbereich einzudringen,
(b) Auftragen der Fremdsubstanz auf den Hautbereich derart, daß ein Teil der Fremdsubstanz in die Haarkanäle eindringt, und
(c) Beleuchtung eines Flecks des Hautbereichs mit den kontaminierten Haarkanälen mit Licht mindestens eines Frequenzbereichs, wobei ein nennenswerter Teil dieses Lichts in die Haut eindringt und von der Fremdsubstanz in den Haarkanälen absorbiert wird, und die optische Absorption der Fremdsubstanz von Licht mindestens eines Frequenzbereichs hoch genug ist, um eine Reaktion hervorzurufen, die die Follikel zerstört oder die die Follikel nährende Haut schädigt.

2. Ein Verfahren gemäß Anspruch 1, wobei die optische Absorption von Licht mindestens einer Frequenz ein Resonanzpeak für die Fremdsubstanz ist.

3. Ein Verfahren gemäß Anspruch 1, wobei die Fremdsubstanz ein Stoff ist, der nicht leicht von den Follikel oder den umgebenden Hautgewebe aufgenommen wird.

4. Ein Verfahren gemäß Anspruch 1, wobei die Fremdsubstanz in einem Medium suspendierte Partikel umfaßt.

5. Ein Verfahren gemäß Anspruch 4, wobei die Partikel eine Größe von 10-20 nm aufweisen.

6. Ein Verfahren gemäß Anspruch 4, wobei die Fremdsubstanz aus einem Öl und einem in diesem Öl suspendierten Absorber besteht.

7. Ein Verfahren gemäß Anspruch 6, wobei der Absorber Kohlepulver ist.

8. Ein Verfahren gemäß Anspruch 6, wobei das Öl Pfirsichöl ist.

9. Ein Verfahren gemäß mindestens einem der Ansprüche 1 bis 4, wobei das die Haare umgebende Hautgewebe geschädigt wird, um die Zerstörung der Follikel im Laufe eines Zeitraumes von einigen Tagen hervorzurufen.

10. Ein Verfahren gemäß mindestens einem der Ansprüche 1 bis 4, wobei das Licht mindestens eines Frequenzbereichs von einem Laser erzeugt wird.

11. Ein Verfahren gemäß Anspruch 10, wobei das Licht mindestens eines Frequenzbereichs Licht einer Wellenlänge von 1.06 µm umfasst.

12. Ein Verfahren gemäß Anspruch 10, wobei das Licht von einem Laser im nahen Infrarot erzeugt wird.

13. Ein Verfahren gemäß Anspruch 10, wobei das Licht mindestens eines Frequenzbereichs ein Bereich ist, der um 10.6 µm (Wellenlänge) zentriert ist und von einem CO₂-Laser erzeugt wird.

14. Ein Verfahren gemäß mindestens einem der Ansprüche 1 bis 4, wobei der Beleuchtungschritt unter Boebachtung des der Beleuchtung unterworfene Fleck durchgeführt wird und mindestens solange fortgeführt wird, bis die Haare auf dem Fleck sich zu locken beginnen.

15. Ein Verfahren gemäß mindestens einem der Ansprüche 1 bis 4, wobei die Reaktion die Follikel erhitzt und oder das die Follikel nährende Hautgewebe in der Nähe der Fremdsubstanz durch indirekte, induktive Heizung erhitzt.

16. Ein Verfahren gemäß mindestens einem der Ansprüche 1 bis 4, wobei der Fleck eine Fläche von etwa 1 bis 5 cm² aufweist.

17. Ein Verfahren gemäß mindestens einem der Ansprüche 1 bis 4, wobei das Auftragen der Fremdsubstanz das Einreiben der Fremdsubstanz in den Hautbereich einschließt.

18. Ein Verfahren gemäß mindestens einem der Ansprüche 1 bis 4, wobei das Auftragen der Fremdsubstanz das Anlegen von Ultraschall an die Fremdsubstanz einschließt.

19. Ein Verfahren gemäß mindestens einem der Ansprüche 1 bis 4, wobei das Auftragen der Fremdsubstanz das erzwungene Eindringen in die Kanäle einschließt.

20. Ein Verfahren gemäß mindestens einem der Ansprüche 1, 2, 10 oder 16, wobei die Fremdsubstanz einen Farbstoff umfaßt.

21. Ein Verfahren gemäß Anspruch 20, wobei der Farbstoff in der Lage ist, die Follikel anzufärben.

22. Ein Verfahren gemäß mindestens einem der Ansprüche 20 oder 21, wobei das Licht mindestens eines Frequenzbereichs einem Absorptionspeak des Farbstoffes angepaßt ist.

23. Ein Verfahren gemäß mindestens einem der Ansprüche 20, 21 oder 22, wobei der Farbstoff ein handelsübliches Haarfärbemittel umfaßt.

24. Ein Verfahren gemäß mindestens einem der Ansprüche 20 bis 23, wobei der Farbstoff Ausziehtusche umfaßt.

25. Ein Verfahren gemäß mindestens einem der Ansprüche 20 bis 23, wobei das Licht mindestens eines Frequenzbereichs einen Intensitätspeak bei mindestens einer Wellenlänge der ungefähr 694 nm, ungefähr 587 nm, 584 nm, 531 nm aufweist.

26. Ein Verfahren gemäß mindestens einem der Ansprüche 1 bis 4, oder 10 bis 25, weiter das Reinigen des Hautbereichs nach dem Auftragen der Fremdsubstanz und vor der Beleuchtung umfassend.

27. Ein Verfahren gemäß mindestens einem der Ansprüche 1 bis 4, oder 10 bis 25, wobei die Beleuchtung die Beleuchtung mit gepulstem Laserlicht einschließt.

## Revendications

1. Un procédé pour l'élimination, d'une zone de la peau humaine, en dehors d'une procédure chirurgicale, de poils poussant dans des canaux pileux à partir de follicules situés au fond desdits canaux pileux et nourris par le tissu cutané dans l'entourage immédiat desdits follicules, essentiellement sans endommager le tissu cutané sauf ledit tissu cutané dans l'entourage immédiat des follicules, comprenant les étapes de :
a) sélection d'un contaminant présentant une absorption optique d'au moins une bande de fréquence de lumière susceptible de pénétrer dans ladite zone de la peau,
b) application dudit contaminant à ladite zone de la peau de manière telle qu'une certaine portion dudit contaminant s'infiltre dans lesdits canaux pileux, et
c) illumination avec au moins une bande de fréquence de lumière d'un spot dans ladite zone de la peau contenant des canaux pileux contaminés, une portion significative de celle-ci pénétrant dans la peau et étant absorbée par ledit contaminant situé dans les canaux pileux, ladite absorption optique par ledit contaminant à ladite au moins une bande de fréquence de lumière étant suffisamment élevée pour provoquer une réaction qui détruit lesdits follicules ou qui endommage les tissus cutanés nourrissant lesdits follicules.

2. Un procédé selon la revendication 1, dans lequel ladite absorption optique à au moins une fréquence est un pic de résonance pour ledit contaminant.

3. Un procédé selon la revendication 1, dans lequel le contaminant est un matériau qui n'est pas aisément absorbé par les follicules ou le tissu de leur entourage immédiat.

4. Un procédé selon la revendication 1, dans lequel ledit contaminant comprend des particules suspendues dans un milieu.

5. Un procédé selon la revendication 4, dans lequel lesdites particules présentent une taille d'environ 10-20 nm.

6. Un procédé selon la revendication 4, dans lequel ledit contaminant est constitué d'une huile et d'un absorbant suspendu dans ladite huile.

7. Un procédé selon la revendication 6, dans lequel ledit absorbant est de la poudre de carbone.

8. Un procédé selon la revendication 6, dans lequel ladite huile est de l'huile de pêche.

9. Un procédé selon l'une quelconque des revendications 1 à 4, dans lequel le tissu entourant les poils est endommagé dans le but de provoquer la destruction des follicules au cours d'une période de quelques jours.

10. Un procédé selon l'une quelconque des revendications 1 à 4, dans lequel ladite au moins une bande de fréquence de lumière est produite par un laser.

11. Un procédé selon la revendication 10, dans lequel ladite au moins une bande de fréquence de lumière comprend de la lumière d'une longueur d'onde d'environ 1.06 microns.

12. Un procédé selon la revendication 10, dans lequel ladite lumière est produite par un laser émettant dans l'infrarouge proche.

13. Un procédé selon la revendication 10, dans lequel ladite au moins une bande de fréquence de lumière est une bande centrée autour de 10.6 microns (longueur d'onde) et est produite par un laser CO₂.

14. Un procédé selon l'une quelconque des revendications 1 à 4, dans lequel ladite étape d'illumination est réalisée tout en observant le spot de peau soumise à l'illumination et en continuant ladite illumination au moins jusqu'à ce que les poils sur l'endroit de la peau commencent à se boucler.

15. Un procédé selon l'une quelconque des revendications 1 à 4, dans lequel ladite réaction chauffe les follicules ou chauffe le tissu cutané nourrissant lesdits follicules à proximité du contaminant par chauffage indirect par conduction.

16. Un procédé selon l'une quelconque des revendications 1 à 4 ou 10, dans lequel ledit spot présente une superficie d'environ 1 à 5 cm².

17. Un procédé selon l'une quelconque des revendications 1 à 4, dans lequel l'application dudit contaminant comprend le massage dudit contaminant sur ladite zone de la peau.

18. Un procédé selon l'une quelconque des revendications 1 à 4, dans lequel l'application dudit contaminant comprend l'application d'ultrasons au contaminant sur la peau.

19. Un procédé selon l'une quelconque des revendications 1 à 4, dans lequel l'application dudit contaminant comprend la pénétration forcée du contaminant dans les canaux.

20. Un procédé selon l'une quelconque des revendications 1, 2, 10 ou 16, dans lequel le contaminant comprend un colorant.

21. Un procédé selon la revendication 20, dans lequel ledit colorant est susceptible de colorer les follicules.

22. Un procédé selon l'une quelconque des revendications 20 ou 21, dans lequel ladite au moins une bande de fréquence correspond à un pic d'absorption dans ledit colorant.

23. Un procédé selon l'une quelconque des revendications 20, 21 ou 22, dans lequel le colorant comprend un colorant du commerce pour les cheveux.

24. Un procédé selon l'une quelconque des revendications 20 à 23, dans lequel ledit colorant comprend de l'encre de Chine.

25. Un procédé selon l'une quelconque des revendications 20 à 23, dans lequel ladite au moins une bande de fréquence comprend un pic d'intensité à une longueur d'onde d'au moins l'un parmi environ 694 nm, environ 587 nm, 584 nm et 531 nm.

26. Un procédé selon l'une quelconque des revendications 1 à 4, ou 20 à 25, comprenant en outre le nettoyage de ladite zone de la peau après l'application dudit contaminant et avant l'illumination.

27. Un procédé selon l'une quelconque des revendications 1 à 4, ou 20 à 25, dans lequel ladite illumination comprend l'illumination par des impulsions lumineuses émises par laser.
